# EUROPEAN PATENT SPECIFICATION

(11) **EP 0 682 946 B1**
(45) Date of publication and mention of the grant of the patent: **19.07.2000**
(21) Application number: 95303203.4
(22) Date of filing: 12.05.1995
(51) Int. Cl.: A61K 9/48, A61K 31/12, A61K 31/77, A61K 35/78

(54) **Laxative compositions**
Abführmittel
Compositions à action laxative

(30) Priority: 19.05.1994 GB 9410103
(43) Date of publication of application: 22.11.1995
(73) Proprietor: Euro-Celtique S.A., Luxemburg (LU)
(72) Inventor: Brown, Adrian, Swaversey, Cambridge CB4 5RZ (GB); Knott, Trevor John, Bishops Stortford, Hertfordshire CM234DG (GB); Leslie, Stewart Thomas, Cambridge CB2 2RA (GB); Miller, Ronald Brown, Basle 4059 (CH); Prater, Derek Allan, Milton, Cambridge CB4 4YS (GB)
(74) Representative: Ruffles, Graham Keith

(56) References cited:
- EP-A- 0 642 786
- WO-A-93/23022
- FR-A- 2 336 922
- DATABASE WPI Week 9529 Derwent Publications Ltd., London, GB; AN 95-215777 & CN-A-1 085 794 (Q. ZENG) , 27 April 1994
- DATABASE WPI Week 9215 Derwent Publications Ltd., London, GB; AN 92-120851 & JP-A-04 066 531 (TAKADA SEIYAKU KK) , 2 March 1992
- DATABASE WPI Week 8947 Derwent Publications Ltd., London, GB; AN 89-345127 & JP-A-01 258 619 (TOYO CAPSULE KK) , 16 October 1989

## Description

This invention is concerned with improvements in and relating to laxative compositions and the manufacture thereof.

Orally administrable compositions containing a stool softener in combination with a stimulant laxative are known in the form of suspensions containing the active ingredients in appropriate liquid carriers for oral administration, for example Co-danthramer suspension (Danthron/Poloxamer 185) sold by Napp Laboratories Limited in the United Kingdom.

Conventionally a solid unit dosage form is prepared by compressing powders into tablets or encapsulating powders into hard gelatin capsules. To achieve satisfactory powder flow and either compression or encapsulation properties, it is conventionally necessary to formulate a product with excipients such as diluents, compression aids, binders, flow aids and lubricants. This is especially true when one or more of the active ingredients has undesirable physicochemical properties, for example is waxy and/or sticky, which make incorporating the ingredient in a conventional dosage form particularly difficult. Furthermore to ensure that the resulting dosage unit will break up and dissolve after administration it is usually necessary to add a disintegrant. These excipient materials add considerably to the volume of the dosage form, and may limit the amount of active ingredient(s) which may be incorporated in a unit dosage form of a size and volume such as to ensure acceptance by a patient. A general discussion A pharmaceutical Formulation can be found in Remington's Pharmaceutical Sciences 16^{th} Edition (1980), Mack Publishing Company.

EP-A 642,786 published 15 March 1995 describes a method for the manufacture of a laxative composition.

It has now been found unexpectedly, in accordance with the present invention, that a surprisingly high dose of a laxative composition can be incorporated in a small size of hard gelatin capsule by forming a stimulant laxative and a stool softener into multiparticulates and filling these into capsules. The resultant composition thus provides an unexpectedly high unit dose of the laxative composition in a pharmaceutical form which is of a size and shape for elegant, convenient and reliable administration to patients.

According to one aspect of the invention we provide a solid unit dosage form comprising a hard gelatin capsule containing a plurality of multiparticulates each comprising a stimulant laxative and a stool softener.

Preferably the capsule is of size 0 to 3.

Suitable stimulant laxatives for use in the dosage forms of the invention include Danthron(1,8-dihydroxyanthraquinones), Bisacodyl, Casanthranol, Cascara, Sennosides and sodium picosulfate, Danthron is particularly preferred.

Preferably the stool softners are those used in amounts which would normally preclude oral unit dosage with a preparation of convenient size, such as the so-called Poloxamers, (polyoxyethylene-polyoxypropylene block copolymers) especially Poloxamer 188 which is normally dosed at about 200mg to 1000mg per dose. Poloxamer 188 is the preferred stool softening agent for use in accordance with the invention. Other pharmaceutically acceptable stool softening laxatives may be used provided their melting point, is in the range - 30 to 90°C, preferably 40 to 80°C.

A suitable dosage form according to the invention is e.g. a size 2 or 3 capsule containing 20-30mg danthron and 180 to 220mg of a poloxamer, or e.g. a size 0 capsule containing 32-43mg danthron and 480-520mg of poloxamer.

The weight ratio of stool softener to stimulant laxative will vary depending upon the specific nature of the two components and the desired intended effect. When the stool softener is Poloxamer 188 and the stimulant laxative is Danthron the weight ratio of stool softener to stimulant laxative is suitably from 2:1 to 20:1, preferably from 4:1 to 15:1 and most preferably from 8:1 to 13.3:1.

According to another aspect of the invention there is provided a process for preparing a laxative, solid oral dosage form which comprises
(a) mechanically working a particulate stimulant laxative agent with a particulate, fusible stool softener, in a mixer at a speed and temperature sufficient to melt or soften the stool softener and create particles comprising stool softener containing the particles of laxative agent dispersed therein;
(b) milling the resulting agglomerates to obtain particles within a desired size range,
(c) blending the particles with lubricant and/or glidant.
(d) filling the particles into a hard gelatin capsule.

If desired other excipients such as disintegrants (e.g. crosscarmellose sodium, crosspovidone, sodium starch glycolate) which may modify performance of the dosage unit may be included in step (a).

Stage (a) of the process may be carried out in conventional high speed mixers with a standard stainless steel interior, e.g. a Collette Vactron 75 or equivalent mixer. The mixture is processed until a bed temperature above 40°C is achieved and the resulting mixture acquires a cohesive granular texture, with particle sizes ranging from about 1-3mm to fine powder in the case of non-aggregated original material. Such material, in the case of the embodiments described below, has the appearance of agglomerates which upon cooling below 40°C have structural integrity and resistance to crushing between the fingers. At this stage the agglomerates are of an irregular size, shape and appearance.

The agglomerates are preferably allowed to cool. The temperature to which they cool is not critical and a temperature in the range room temperature to 41°C may be conveniently used.

The agglomerates are broken down by any suitable means which will comminute oversize agglomerates and produce a mixture of powder and small particles preferably with a diameter under 2mm. It is currently preferred to carry out the classification using a Jackson Crockatt granulator using a suitable sized mesh, or a Comil with an appropriate sized screen. We have found that if too small a mesh size is used in the aforementioned apparatus the agglomerates melting under the action of the beater or impeller will clog the mesh and prevent further throughput of mixture, thus reducing yield.

In order to ensure uniform energy input into the ingredients in the high speed mixer it is preferred to supply at least part of the energy by means of microwave energy.

Energy may also be delivered through other means such as by a heating jacket or via the mixer impeller and chopper blades.

The resulting particles may be used to prepare dosage units e.g. capsules in manners known per se.

In order that the invention may be well understood the following Examples and Comparative Examples are given by way of illustration only.

### Example 1

125g of danthron followed by 1000g poloxamer 188 were added to the bowl of a Collette Gral 10 mixer/granulator (10 litre) pre-heated to approximately 45°C.

The mixer and granulator were operated at 350rpm and 1500rpm. The process temperature was increased to 47°C over 17 minutes and the processing continued until a multiparticulate product had formed.

The product was removed from the Collette bowl and cooled to room temperature. The multiparticulates were then passed through a 1.40mm screen using a Jackson Crockett granulator.

The multiparticulates were blended with 2% w/w magnesium stearate for 5 minutes in a Y-cone blender then encapsulation carried out using a Zanasi AZ5 capsule filling machine. The capsules used were hard gelatin capsules Size 2 (volume 0.37ml).

The size 2 capsules receive multiparticulates giving the dosage per capsule as follows;

| | **Danthron** | **Poloxamer 188** |
|---|---|---|
| Size 2 | 25mg | 200mg |

### Comparative Example 1

| Co-danthramer Capsule 25mg/200mg | |
|---|---|
| | mg/capsule |
| Danthron B.P. | 25.0 |
| Poloxamer 188 U.S.N.F. | 200 |
| Total | 225 |

Blend powdered poloxamer 188 and the danthron in a mixer until a uniform blend is obtained. Fill powder into size 1 hard gelatin capsule (volume 0.5 ml). The density of the contents of the capsule shell, prepared according to the comparative example is approximately 0.45g/ml. Therefore by preparing capsules by conventional powder filling the normal dose of co-danthramer can only be achieved by administering one or two size 1 capsules even in the absence of excipients which would be required to enable the powder blend to be filled on a high speed capsule filling machine.

### Example 2

Example 1 was repeated but using 75g of danthron and 1000g poloxamer 188. The capsules used were hard gelatin capsules Size 0 (volume 0.68ml).

The size 0 capsules receive multiparticulates giving the dosage per capsule as follows;

| | Danthron | Poloxamer 188 |
|---|---|---|
| Size 0 | 37.5mg | 500mg |

### Comparative Example 2

| Strong Co-danthramer capsule 37.5 mg/500mg | |
|---|---|
| | mg/capsule |
| Danthron B.P. | 37.5 |
| Poloxamer 188 U.S.N.F. | 500 |
| Total | 537.5 |

Blend powdered poloxamer 188 and the danthron in a mixer until a uniform blend is obtained. fill powder blend into size 000 hard gelatin capsule (volume 1.37ml). The density of the contents of the capsule shell, prepared according to the comparative example is approximately 0.45g/ml. Therefore by preparing capsules by conventional powder filling the normal dose of strong co-danthramer can only be achieved by administering one or two size 000 capsules even in the absence of excipients which would be required to enable the powder blend to be filled on a high speed capsule filling machine. Size 000 capsules are generally regarded as too large for human administration, being utilized for large animal veterinary applications and use of smaller size 0 capsules would necessitate administration of two or four capsules to achieve the normal dose of strong co-danthramer.

Thus it was found that conventional methods of formulation of a stimulant laxative, danthron, and a stool softener, poloxamer 188 yielded an unacceptably large dosage form even before addition of the excipients necessary to allow commercial manufacture.

Preparation of capsules according to the invention, therefore, allows for the manufacture of a laxative composition in unit dosage form without addition of any excipients or other adjuvant apart from lubricant/glident and which furthermore minimizes the volume of the active ingredients to enable achievement of an unexpectedly high dose in a preparation of small physical size.

## Claims

1. A solid, unit dosage form comprising a hard gelatin capsule containing a plurality of multiparticulates each comprising a stimulant laxative and a stool softener.

2. A dosage form according to claim 1, wherein the stimulant laxative is danthron.

3. A dosage form according to claim 1 or claim 2, wherein the stool softener is a poloxamer, preferably Poloxamer 188.

4. A dosage form according to claim 3, which is a size 2 or 3 capsule containing 20-30mg danthron and 180-220mg of a poloxamer.

5. A dosage form according to claim 3, which is a size 0 capsule containing 32-43mg danthron and 480 to 520mg of a poloxamer.

6. A dosage form according to anyone of claims 2 to 5 wherein the weight ratio of poloxamer to danthron is 2:1 to 20:1.

7. A method of preparing a laxative, solid oral dosage form which comprises,
(a) mechanically working a particulate stimulant laxative agent with a particulate, fussible stool softener in a mixer at a speed and temperature sufficient to melt or soften the stool softener and create particles comprising stool softener containing the particles of laxative agent dispersed therein;
(b) milling the resulting agglomerates to obtain particles within a desired size range,
(c) blending the particles with lubricant and/or glidant.
(d) filling the particles into a hard gelatin capsule.

8. A method according to claim 7, wherein the working is carried out in a high shear mixer.

9. A method according to claim 7, wherein during the mechanical working, heat is supplied by microwave radiation.

10. A method according to claim 9, wherein only a part of the heating is supplied by microwave radiation.

11. A method according to claim 7, 8, 9 or 10 wherein the stimulant laxative is danthron.

12. A method according to claim 7, 8, 9, 10 or 11 wherein the stool softener is a poloxamer, preferably Poloxamer 188.

13. A method according to claims 11 and 12, wherein the weight ratio of poloxamer to danthron is from 2:1 to 20:1.

## Patentansprüche

1. Feste, einheitliche Dosierungsform, aufweisend eine Hartgelatinekapsel, enthaltend eine Vielzahl von Multiteilchen, jeweils aufweisend ein stimulierendes Abführmittel und einen Stuhlerweicher.

2. Dosierungsform gemäß Anspruch 1, wobei das stimulierende Abführmittel Danthron ist.

3. Dosierungsform gemäß Anspruch 1 oder Anspruch 2, wobei der Stuhlerweicher ein Poloxamer, vorzugsweise Poloxamer 188, ist.

4. Dosierungsform gemäß Anspruch 3, welche eine Kapsel der Größe 2 oder 3, enthaltend 20-30 mg Danthron und 180-220 mg eines Poloxamers, ist.

5. Dosierungsform gemäß Anspruch 3, welche eine Kapsel der Größe 0, enthaltend 32-43 mg Danthron und 480 bis 520 mg eines Poloxamers, ist.

6. Dosierungsform gemäß einem der Ansprüche 2 bis 5, wobei das Gewichtsverhältnis von Poloxamer zu Danthron 2:1 bis 20:1 beträgt.

7. Verfahren zur Herstellung einer abführenden, festen oralen Dosierungsform, welches umfaßt,
(a) mechanisches Bearbeiten eines teilchenförmigen stimulierenden Abführmittels mit einem teilchenförmigen, schmelzbaren Stuhlerweicher in einem Mischapparat bei einer Geschwindigkeit und Temperatur, die ausreichend sind, um den Stuhlerweicher zu schmelzen oder zu erweichen und einen Teilchen umfassenden Stuhlerweicher, enthaltend die Teilchen des darin dispergierten Abführmittels, zu erzeugen;
(b) Zermahlen der entstehenden Agglomerate, um Teilchen innerhalb eines gewünschten Größenbereichs zu erhalten,
(c) Vermischen der Teilchen mit Schmiermittel und/oder Gleitmittel,
(d) Einfüllen der Teilchen in eine Hartgelatinekapsel.

8. Verfahren gemäß Anspruch 7, wobei die Bearbeitung in einem Mischapparat mit hoher Scherung ausgeführt wird.

9. Verfahren gemäß Anspruch 7, wobei während der mechanischen Bearbeitung durch Mikrowellenstrahlung Wärme geliefert wird.

10. Verfahren gemäß Anspruch 9, wobei nur ein Teil der Erwärmung durch Mikrowellenstrahlung geliefert wird.

11. Verfahren gemäß Anspruch 7, 8, 9 oder 10, wobei das stimulierende Abführmittel Danthron ist.

12. Verfahren gemäß Anspruch 7, 8, 9, 10 oder 11, wobei der Stuhlerweicher ein Poloxamer, vorzugsweise Poloxamer 188, ist.

13. Verfahren gemäß den Ansprüchen 11 und 12, wobei das Gewichtsverhältnis von Poloxamer zu Danthron von 2:1 bis 20:1 beträgt.

## Revendications

1. Forme de dosage unitaire, solide, comprenant une capsule de gélatine dure contenant une pluralité de multiparticules, chacune comprenant un laxatif stimulant et un adoucissant de selles.

2. Forme de dosage suivant la revendication 1, dans laquelle le laxatif stimulant est de la danthrone.

3. Forme de dosage suivant la revendication 1 ou la revendication 2, dans laquelle l'adoucissant de selles est un poloxamère, de préférence le Poloxamer 188.

4. Forme de dosage suivant la revendication 3, qui est une capsule de taille 2 ou 3 contenant 20-30 mg de danthrone et 180-220 mg d'un poloxamère.

5. Forme de dosage suivant la revendication 3, qui est une capsule de taille 0 contenant 32-43 mg de danthrone et 480 à 520 mg d'un poloxamère.

6. Forme de dosage suivant l'une quelconque des revendications 2 à 5, dans laquelle le rapport pondéral du poloxamère sur la danthrone est de 2:1 à 20:1.

7. Procédé pour la préparation d'une forme de dosage solide orale, laxative, qui comprend:
(a) le travail mécanique d'un agent laxatif stimulant particulaire avec un adoucissant de selles fusible, particulaire dans un mélangeur à une vitesse et à une température suffisantes pour fondre ou ramollir l'adoucissant de selles et créer des particules comprenant l'adoucissant de selles contenant les particules d'agent laxatif dispersées à l'intérieur de l'adoucissant;
(b) le broyage des agglomérats résultants pour obtenir des particules dans un domaine de taille désiré;
(c) le mélange des particules avec un lubrifiant et/ou un agent de glissement;
(d) le remplissage des particules dans une capsule de gélatine dure.

8. Procédé suivant la revendication 7, dans lequel le travail est réalisé dans un mélangeur à cisaillement élevé.

9. Procédé suivant la revendication 7, dans lequel, durant le travail mécanique, de la chaleur est alimentée par un rayonnement micro-onde.

10. Procédé suivant la revendication 9, dans lequel seulement une partie du chauffage est alimentée par un rayonnement micro-onde.

11. Procédé suivant la revendication 7, 8, 9 ou 10, dans lequel le laxatif stimulant est de la danthrone.

12. Procédé suivant la revendication 7, 8, 9, 10 ou 11, dans lequel l'adoucissant de selles est un poloxamère, de préférence le Poloxamer 188.

13. Procédé suivant les revendications 11 et 12, dans lequel le rapport pondéral du poloxamère sur la danthrone est de 2:1 à 20:1.
